# EUROPEAN PATENT SPECIFICATION

(11) **EP 1 043 986 B1**
(45) Date of publication and mention of the grant of the patent: **16.04.2003**
(21) Application number: 98966848.8
(22) Date of filing: 23.12.1998
(51) Int. Cl.: A61K 31/135, A61K 47/48

(54) **MIXTURE AND PHARMACEUTICAL COMPOSITION COMPRISING Z-4-HYDROXYTAMOXIFEN AND CYCLODEXTRIN**
GEMISCH UND PHARMAZEUTISCHE ZUBEREITUNG ENTHALTEND Z-4-HYDROXYTAMOXIFEN UND CYCLODEXTRIN
MELANGE ET COMPOSITION PHARMACEUTIQUE COMPRENANT DU Z-4- HYDROXYTAMOXIFENE ET DE LA CYCLODEXTRINE

(30) Priority: 23.12.1997 EP 97122742
(43) Date of publication of application: 18.10.2000
(73) Proprietor: HEXAL AG, D-83607 Holzkirchen (DE)
(72) Inventor: FISCHER, Wilfried, D-83607 Holzkirchen (DE); KLOKKERS, Karin, D-83607 Holzkirchen (DE); SENDL-LANG, Anna, D-83607 Holzkirchen (DE)
(74) Representative: Boeters, Hans Dietrich, Dr.
(86) International application number: EP9808437
(87) International publication number: WO99033451

(56) References cited:
- EP-A- 0 287 690
- R.DOUGLAS ARMSTRONG: "Separation of tamoxifen geometric isomers and metabolites by bonded-phase beta-cyclodextrin chromatography" J.CHROMATOGR.BIOMED.APPL., vol. 414, no. 1, 1987, pages 192-196, XP002107478
- UEKAMA K: "PHARMACEUTICAL APPLICATIONS OF METHYLATED CYCLODEXTRINS" PHARMACY INTERNATIONAL, vol. 6, 1 March 1985, pages 61-65, XP002053303

## Description

### Field of the invention

The present invention relates to new pharmaceutical compositions of Z-4-hydroxytamoxifen having improved solubility and stability.

### Background of the invention

4-hydroxytamoxifen [1-(p-β-dimethylaminoethoxyphenyl)-1-(p-hydroxyphenyl)-2-phenylbut-1-en ] is a metabolite of tamoxifen, a well known cytostatic antiestrogen. Tamoxifen, a nonsteroidal antiestrogen, is primarily used for the treatment of hormone dependent breast cancer. Special tests have shown, that 4-hydroxytamoxifen is more potent in treating breast cancer than tamoxifen ( Jordan V.C. et al.; J. Endocr. (1977), 75, 305-316). There exist two isomers of 4-hydroxytamoxifen, the Z- and E-isomer. The Z-isomer is the active component for treating breast cancer but the stability of this isomer is very low. The Z-E-isomerization of 4-hydroxytamoxifen takes place primarily in solution and an equilibrium of the two isomers is reached after a short time. The E-isomer shows estrogen activity and this activity is a contrast to the Z-isomer. For the treatment of breast cancer the content of the E-isomer has to be less than one percent to guarantee a successful treatment. A further problem of Z-4-hydroxytamoxifen is its poor aqueous solubility. To get a solution for an injection, the 4-hydroxytamoxifen has to be soluble in an aqueous medium. Therefore it exists a strong need for parenteral formulations of the anticancer antiestrogens. Injectable high concentration 4-hydroxytamoxifen formulations will have important clinical benefit
1) in the attempt to reach high concentrations in tissue. This is necessary especially when combinations of 4-hydoxytamoxifen with cytotoxic drugs are given to a patient. High plasma concentrations are more effective in reversing multidrug resistance than low concentrations. An injectable formulation enables high peak concentrations in blood and tissues without exposing the patient to long-term treatment;
2) when given locally into a tumor . This enables a high and efficacious concentration in the tumor to be achieved.

DE 28 07 599 discloses the use of 4-hydroxytamoxifen for the treatment of breast cancer. EP 0 287 690 discloses the stabilization of the Z-isomer of 4-hydroxytamoxifen by forming an adduct with a solvent. This process of stabilization is very time consuming and complicated. The preferred solvents forming the adduct are acetone, butanone, acetylacetone, mesityloxide, acetic acid ethylester and acetoacetic acid ethylester. These adducts are not suitable for parenteral administration. EP 0 616 529 discloses parenteral preparations of antineoplasmic agents like tamoxifen, toremifen, desmethyltamoxifen or desmethyltoremifen in form of stable emulsions or liposomes to ensure improved solubility.

The problem of a stable solution of Z-4-hydroxytamoxifen with a E-isomer content of less than one percent and an easy and cheap process of stabilization and the possibility of solubilization of a therapeutic effective amount of Z-4-hydroxytamoxifen in an aqueous solution has not been solved until now.

### Summary of the invention

Main object of the present invention is to provide new compositions of Z-4-hydroxytamoxifen with improved stability and aqueous solubility.

It has now surprisingly been found, that Z-4-hydroxytamoxifen can be stabilized by complexation with cyclodextrin. It has further been found that the solubility of the Z-4-hydroxytamoxifen in aqueous solution can be improved by forming a Z-4-hydroxytamoxifen/cyclodextrin complex. Therefore it is now possible to produce special dosage forms containing the Z-4-hydroxytamoxifen/ cyclodextrin complex, resulting from the improved solubility and stability.

Thus, the invention concerns a mixture consisting of or comprising Z-4-hydroxytamoxifen and cyclodextrin.

According to the invention Z-4-hydroxytamoxifen may be used per se or as a pharmaceutically acceptable salt thereof, such as hydrochloride, sulfate, phosphate, acetate, tartrate or citrate.

The cyclodextrin may be a pharmaceutically acceptable cyclodextrin or a cyclodextrin which is used for pharmaceutical preparations.

Thus, the cyclodextrin may be at least one component selected from the group consisting of gamma-cyclodextrin, β-cyclodextrin, dimethyl-β-cyclodextrin, hydroxypropyl-β-cyclodextrin, random methylated β-cyclodextrin and maltosyl-β-cyclodextrin.

Especially, the cyclodextrin is gamma-cyclodextrin.

Further, the mixture according to the invention may comprise or contain cyclodextrin in such an amount that the stability and solubilty of Z-4-hydroxytamoxifen in aqueous media is improved.

Further, the mixture according to the invention may have a molar ratio of Z-4-hydroxytamoxifen to cyclodextrin within the range of 1 : 1 to 1 : 50, preferably 1 : 1 to 1 : 20 and especially 1 : 1 to 1 : 10.

Further, the mixture according to the invention may be obtainable by
- dissolving or suspending Z-4-hydroxytamoxifen and cyclodextrin in a liquid medium and
- bringing the solution or suspension to dryness.

The liquid medium may be an aqueous medium and especially water. Further, the liquid medium may be subjected to ultrasonification during said dissolving or suspending or thereafter.

Further, the resulting solution or suspension may be filtrated before bringing it to dryness, especially filtrated through a membrane filter of an average pore size of about 0.45 x 10⁻⁶ m or less.

Further, the resulting optionally ultrasonicated and optionally filtrated solution or suspension may be chilled, the resulting precipitate separated and the separated precipitate brought to dryness.

Further, the invention concerns a pharmaceutical composition comprising or consisting of a mixture according to the invention, optional auxiliary agents and optionally at least one additional active ingredient.

The pharmaceutical composition according to the invention may be tailored for an administration selected from the group consisting of parenteral, oral, rectal, transdermal and ophthalmic administration.

Especially, the pharmaceutical preparation according to the invention may be tailored for parenteral administration.

Thus, the pharmaceutical preparation according to the invention may comprise or consist of a solution or suspension of a mixture according to the invention in an aqueous medium. According to a preferred embodiment, the aqueous medium is water or physiological saline.

Thus, the problem underlying the invention is solved by a pharmaceutical composition comprising or consisting of
■ Z-4-hydroxytamoxifen as active ingredient and as excipients
■ at least one cyclodextrin.

As regards auxiliary agents to be used for the preparation of compositions according to the invention, reference can be made to the state of the art such as DE 28 07 599, EP 0 287 690 or EP 0 616 529.

In order to describe the invention more specifically but without intending to limit the scope of the invention in any way the following examples are presented:

### Example 1:

50 mg (0.129 mmol) 4-hydroxytamoxifen (MW 387.5) and 1,0 g (0.771 mmol) γ-cyclodextrin (MW 1297) were dissolved in 35 ml physiological saline (phosphat buffer pH 7). An opalescent solution was obtained by ultrasonication for about 5 minutes. The content of 4-hydroxytamoxifen in the solution was 5%.After filtration through a 0,45 µm membrane filter the clear filtrate was chilled by a dry ice/ ethanol mix and the complex was isolated by freeze drying. The 4-hydroxytamoxifen content of the complex is 3,33 % ± 0,1 % by weight.

### Example 2:

155,2 mg 4-hydroxytamoxifen/γ-cyclodextrin complex according to **example 1** were dissolved in 50 ml distilled water. A clear solution was immediately obtained. The pH value of the solution was around pH 7,1. The solution was filtered through a 0,22 µm membrane filter. The solution was filled in flasks. The samples were stored for eight days under sterile conditions at room temperature (25 °C ) and at 4 °C. The content of the solution was determined by diluting the solution five times with 50 % ethanol and then analyzed spectrometrically. The 4-hydroxytamoxifen content of the standard (25 °C and no storage time ) was 98,5 µg/ ml. The content of 4-hydroxytamoxifen was 98,1 µg/ ml at 25 °C after eight days of storage. After eight days of storage at 4 °C the content of 4-hydroxytamoxifen was 98,5 µg/ml.

### Example 3:

A stability test was performed for 24 hours at room temperature. The 4-hydroxytamoxifen/γ-cyclodextrin complex according to **example 1** was dissolved in water and exposed to day light at room temperature. The E/Z isomer ratio was analyzed by capillary electrophoresis (CE). The results are shown in **table 1.** The amount of the E-isomer was below the detection limit demonstrating the stabilizing effect of the cyclodextrin complex in solution.

**Table 1:**

| 4-Hydroxytamoxifen/ γ-cyclodextrin complex stored in solution at room temperature | | |
|---|---|---|
| **Storage time in hours** | **Content of Z-4-hydroxytamoxifen in µg/ ml*** | **Ratio r** _{**e/z**} |
| 0 | 104,0 ± 0,3 | 0 |
| 1 | 102,7 ± 0,7 | 0 |
| 2 | 102,2 ± 0,2 | 0 |
| 3 | 109,4 ± 0,4 | 0 |
| 4 | 104,3 ± 0,3 | 0 |
| 5 | 105,4 ± 0,4 | 0 |
| 6 | 100,9 ± 0,5 | 0 |
| 7 | 104,9 ± 0,9 | 0 |
| 8 | 99,0 ± 0,1 | 0 |
| 24 | 102,1 ± 0,2 | 0 |

| | | |
|---|---|---|
| *calculation based on a 5-point calibration, n=2 | | |

### Example 4:

A 10-day stability test in water and in 96 % ethanol at room temperature and exposure to normal day light, using 4-hydroxytamoxifen and the 4-hydrorytamoxifen/ γ-cyclodextrin complex prepared according to **example 1** was performed. The change of E/Z isomer ratio was analyzed with capillary electrophoresis. The results are shown in **table 2**.

**Table 2:**

| Calculated isomer ratios during 10-days storage | | | |
|---|---|---|---|
| Sample | In water | Ratio r_{e/z} | Efficiency value* |
| 4-Hydroxytamoxifen | n. a. ** | 0,223 | 4,65 |
| 4-Hydroxytamoxifen/γ-cyclodextrin complex | 0 | 0,048 | |

| | | | |
|---|---|---|---|
| * ratio of uncomplexed and complexed 4-hydroxytamoxifen r_{e/z} values | | | |
| ** n.a. - not applicable, it is not soluble in water | | | |

The 4-hydroxytamoxifen/ γ-cyclodextrin complex dissolved in water showed no detectable change in isomer ratio (r_{e/z} = 0). In the tested samples no detectable amount of E isomer was found.
A small change of isomer ratio (r_{e/z} = 0,048) was found using 96 % ethanol as solvent for the 4-hydroxytamoxifen/ γ-cyclodextrin complex.
4-Hydroxytamoxifen dissolved in 96 % ethanol showed a considerable change in isomer ratio (r_{e/z} = 0,223).
The change of isomer ratio in water was not investigated due to the insolubility of 4-hydroxytamoxifen in water.

### Example 5:

A twelve months stability test of the solid complex according to example 1 was performed under controlled conditions (25 °C, 60 % relative humidity (R. H.)) and a control sample in the refrigerator (4 °C, no controlled humidity). The E/Z isomer ratio was analyzed by capillary electrophoresis. The results are shown in **table 3**.

**Table 3:**

| | 4-Hydroxytamoxifen/ γ-cyclodextrin complex (stored as solid complex) controlled conditions at 25 °C, 60 % R.H. | | | 4-Hydroxytamoxifen/ γ-cyclodextrin complex (stored as solid complex) control sample at 4 °C, R. H. not controlled | | |
|---|---|---|---|---|---|---|
| **storage time in months** | **content of Z-4-hydroxytamoxifen in % based on dry weight** | **ratio r** _{**e/z**} | **water content in %** | **content of Z-4-hydroxytamoxifen in % based on dry weight** | **ratio r** _{**e/z**} | **water content in %** |
| 0 | 3,18 ± 0,11 | 0* | 6,67 ± 0,07 | 3,18 ± 0,11 | 0* | 6,67 ± 0,07 |
| 3 | 3,4 ± 0,36 | 0* | 11,94 ± 0,16 | n.m.** | n.m. ** . | n.m.** |
| 6 | 3,56 ± 0,11 | 0* | 13,39 ± 0,17 | 3,52 ± 0,11 | 0* | 8,02 ± 0,05 |
| 12 | 3,51 ± 0,04 | 0* | 15,23 ± 0,07 | 3,53 ± 0,06 | 0* | 9,91 ± 0,09 |

| | | | | | | |
|---|---|---|---|---|---|---|
| *E isomer not detectable | | | | | | |
| ** not measured | | | | | | |

## Claims

1. Pharmaceutical mixture comprising or consisting of Z-4-hydroxitamoxifen and at least one cyclodextrin.

2. Mixture according to claim 1, wherein the cyclodextrin is a pharmaceutically acceptable cyclodextrin or a cyclodextrin which is used for pharmaceutical preparations.

3. Mixture according to claim 1 or 2, wherein the cyclodextrin is at least one component selected from the group consisting of gamma-cyclodextrin, β-cyclodextrin, dimethyl-β-cyclodextrin, hydroxypropyl-β-cyclodextrin, random methylated β-cyclodextrin and maltosyl-β-cyclodextrin.

4. Mixture according to claim 3, wherein the cyclodextrin is gamma-cyclodextrin.

5. Mixture according to any of the preceding claims, wherein the mixture comprises or contains cyclodextrin in such an amount that the stability and solubilty of Z-4-hydroxytamoxifen in aqueous media is improved.

6. Mixture according to any of the preceding claims and especially claim 5, wherein the molar ratio of Z-4-hydroxytamoxifen to cyclodextrin is within the range of 1 : 1 to 1 : 50, preferably 1 : 1 to 1 : 20 and especially 1 : 1 to 1 : 10.

7. Mixture according to any of the preceding claims, wherein the mixture is obtainable by
- dissolving or suspending Z-4-hydroxytamoxifen and cyclodextrin in a liquid medium and
- bringing the solution or suspension to dryness.

8. Mixture according to claim 7, wherein the liquid medium is an aqueous medium and especially water.

9. Mixture according to claim 7 or 8, wherein the liquid medium is subjected to ultrasonification during said dissolving or suspending or thereafter.

10. Mixture according to any of claims 7 to 9, wherein the resulting solution or suspension is filtrated before bringing it to dryness, especially filtrated through a membrane filter of an average pore size of about 0.45 x 10⁻⁶ m or less.

11. Mixture according to any of claims 7 to 10, wherein the resulting optionally ultrasonicated and optionally filtrated solution or suspension is chilled, the resulting precipitate is separated and the separated precipitate is brought to dryness.

12. Pharmaceutical composition comprising or consisting of a mixture according to any of the preceding claims, optional auxiliary agents and optionally at least one additional active ingredient.

13. Pharmaceutical composition according to claim 12 tailored for an administration selected from the group consisting of parenteral, oral, rectal, transdermal and ophthalmic administration.

14. Pharmaceutical composition according to claim 13, wherein the composition is tailored for parenteral administration.

15. Pharmaceutical composition according to claim 14, wherein the composition comprises or consists of a solution or suspension of a mixture according to any of claims 1 to 11 in an aqueous medium.

16. Pharmaceutical composition according to claim 15, wherein the aqueous medium is water or physiological saline.

## Patentansprüche

1. Pharmazeutische Mischung, umfassend oder bestehend aus Z-4-Hydroxytamoxifen und mindesten einem Cyclodextrin.

2. Mischung nach Anspruch 1, wobei das Cyclodextrin ein pharmazeutisch verträgliches Cyclodextrin oder ein Cyclodextrin ist, das für pharmazeutische Zubereitungen verwendet wird.

3. Mischung nach Anspruch 1 oder 2, wobei das Cyclodextrin mindestens eine Komponente der Gruppe ist, die durch gamma-Cyclodextrin, beta-Cyclodexrin, Dimethyl-beta-cyclodextrin, Hydroxypropyl-beta-cyclodextrin, statistisch methyliertes beta-Cyclodextrin und Maltosyl-beta-cyclodextrin gebildet wird.

4. Mischung nach Anspruch 3, wobei das Cyclodextrin gamma-Cyclodextrin ist.

5. Mischung nach einem der vorhergehenden Ansprüche, wobei die Mischung Cyclodextrin in einer derartigen Menge umfaßt oder enthält, dass die Stabilität und Löslichkeit von Z-4-Hydroxytamoxifen in wässerigem Medium verbessert wird.

6. Mischung nach einem der vorhergehenden Ansprüche und insbesondere nach Anspruch 5, wobei das Molverhältnis von Z-4-Hydroxytamoxifen zu Cyclodextrin im Bereich von 1:1 bis 1:50, vorzugsweise 1:1 bis 1:20 und insbesondere 1:1 bis 1:10 liegt.

7. Mischung nach einem der vorhergehenden Ansprüche, wobei die Mischung dadurch erhältlich ist, dass man
- Z-4-Hydroxytamoxifen und Cyclodextrin in einem flüssigem Medium löst oder suspendiert und
- die Lösung oder Suspension zur Trockne bringt.

8. Mischung nach Anspruch 7, wobei das flüssige Medium ein wässriges Medium und insbesondere Wasser ist.

9. Mischung nach Anspruch 7 oder 8, wobei das flüssige Medium einer Ultrabeschallung beim Lösen oder Suspendieren oder danach unterworfen worden ist.

10. Mischung nach einem der Ansprüche 7 bis 9, wobei die resultierende Lösung oder Suspension filtriert worden ist, bevor sie zur Trockne gebracht worden ist, insbesondere durch ein Membranfilter einer mittleren Porengröße von etwa 0,45 x 10⁻⁶ m oder weniger.

11. Mischung nach einem der Ansprüche 7 bis 10, wobei die resultierende gegebenenfalls ultrabeschallte und gegebenenfalls abfiltrierte Lösung oder Suspension abgeschreckt, der resultierende Niederschlag abgetrennt und der abgetrennte Niederschlag zur Trockne gebracht worden ist.

12. Pharmazeutische Zusammensetzung, umfassend oder bestehend aus einer Mischung gemäß einem der vorhergehenden Ansprüche, gegebenenfalls Hilfsmitteln und gegebenenfalls mindestens einem zusätzlichem Wirkstoff.

13. Pharmazeutische Zusammensetzung nach Anspruch 12, zubereitet für eine Verabreichung, ausgewählt unter parenteraler, oraler, rektaler, transdermaler und ophthalmischer Verabreichung.

14. Pharmazeutische Zusammensetzung nach Anspruch 13, wobei die Zusammensetzung zur parenteralen Verabreichung zubereitet ist.

15. Pharmazeutische Zusammensetzung nach Anspruch 14, wobei die Zusammensetzung eine Lösung oder Suspension einer Mischung gemäß einem der Ansprüche 1 bis 11 in einem wässerigem Medium umfaßt oder daraus besteht.

16. Pharmazeutische Zusammensetzung nach Anspruch 15, wobei das wässerige Medium Wasser oder eine physiologische Kochsalzlösung ist.

## Revendications

1. Mélange pharmaceutique comprenant ou consistant en du Z-4-hydroxy-tamoxifène et au moins une cyclodextrine.

2. Mélange conforme à la revendication 1, dans lequel la cyclodextrine est une cyclodextrine admissible en pharmacie ou une cyclodextrine employée pour des préparations pharmaceutiques.

3. Mélange conforme à la revendication 1 ou 2, dans lequel la cyclodextrine est au moins un composant choisi dans l'ensemble formé par les γ-cyclodextrine, β-cyclodextrine, diméthyl-β-cyclodextrine, hydroxypropyl-β-cyclodextrine, β-cyclodextrine aléatoirement méthylée et maltosyl-β-cyclodextrine.

4. Mélange conforme à la revendication 3, dans lequel la cyclodextrine est de la γ-cyclodextrine.

5. Mélange conforme à l'une des revendications précédentes, lequel mélange comprend ou contient une cyclodextrine en une quantité telle que la stabilité et la solubilité du Z-4-hydroxytamoxifène dans les milieux aqueux sont améliorées.

6. Mélange conforme à l'une des revendications précédentes, en particulier à la revendication 5, dans lequel le rapport molaire du Z-4-hydroxytamoxifène à la cyclodextrine se situe dans l'intervalle allant de 1/1 à 1/50, de préférence de 1/1 à 1/20, et en particulier de 1/1 à 1/10.

7. Mélange conforme à l'une des revendications précédentes, lequel mélange peut être obtenu par
- dissolution ou mise en suspension du Z-4-hydroxytamoxifène et de la cyclodextrine dans un milieu liquide,
- et dessiccation de la solution ou de la suspension.

8. Mélange conforme à la revendication 7, dans lequel le milieu liquide est un milieu aqueux, en particulier de l'eau.

9. Mélange conforme à la revendication 7 ou 8, dans lequel le milieu liquide subit une ultrasonication pendant ou après ladite dissolution ou mise en suspension.

10. Mélange conforme à l'une des revendications 7 à 9, pour lequel on filtre la solution ou suspension obtenue avant de la dessécher, et en particulier, on la filtre en la faisant passer à travers une membrane filtrante dont les pores présentent une taille moyenne inférieure ou égale à environ 0,45.10⁻⁶ m.

11. Mélange conforme à l'une des revendications 7 à 10, pour lequel on refroidit la solution ou suspension obtenue, qui a éventuellement subi une ultrasonication et qui a éventuellement été filtrée, puis on isole le précipité formé, et l'on dessèche le précipité isolé.

12. Composition pharmaceutique comprenant ou consistant en un mélange conforme à l'une des revendications précédentes, ainsi que, éventuellement, des agents auxiliaires et eventuellement au moins un ingrédient actif supplémentaire.

13. Composition pharmaceutique conforme à la revendication 12, adaptée en vue d'une administration par une voie choisie parmi les voies parentérale, orale, rectale, transdermique et oculaire d'administration.

14. Composition pharmaceutique conforme à la revendication 13, qui est une préparation adaptée en vue d'une administration par voie parentérale.

15. Composition pharmaceutique conforme à la revendication 14, qui est une préparation comprenant ou consistant en une solution ou suspension d'un mélange conforme à l'une des revendications 1 à 11 dans un milieu aqueux.

16. Composition pharmaceutique conforme à la revendication 15, dans laquelle le milieu aqueux est de l'eau ou du soluté physiologique.
